# EUROPÄISCHE PATENTSCHRIFT

(11) **EP 0 769 488 B1**
(45) Veröffentlichungstag und Bekanntmachung des Hinweises auf die Patenterteilung: **14.07.1999**
(21) Anmeldenummer: 96114580.2
(22) Anmeldetag: 12.09.1996
(51) Int. Cl.: C07C 201/08, C07C 205/45

(54) **Verfahren zur Herstellung von 3-Chlor-3'-nitro-4'methoxybenzophenon**
Process for the preparation of 3-chloro-3'-nitro-4' methoxybenzophenone
Procédé pour la préparation du chloro-3-nitro-3'-méthoxy-4'-benzophénone

(30) Priorität: 25.09.1995 DE 19535500
(43) Veröffentlichungstag der Anmeldung: 23.04.1997
(73) Patentinhaber: BAYER AG, 51368 Leverkusen (DE)
(72) Erfinder: Paetz, Klaus-Christian, Dr., 51399 Burscheid-Hilgen (DE); Fiege, Helmut, Dr., 51373 Leverkusen (DE); Kissener, Wolfram, Dr., 53819 Neunkirchen-Seelscheid (DE)

(56) Entgegenhaltungen:
- EP-A- 0 023 557
- ARZNEIMITTEL FORSCHUNG DRUG RESEARCH., Bd. 28, Nr. 4, 1978, AULENDORF DE, Seiten 586-594, XP000195817 A. H. M. RAEYMAEKERS ET AL.: "Synthesis and Anthelminthic Activity...."
- CHEMICAL ABSTRACTS, vol. 107, no. 25, 21.Dezember 1987 Columbus, Ohio, US; abstract no. 236236k, Seite 741; Spalte 2; XP002022340 & RO 91 633 A (INTREPRINDEREA DE MEDICAMENTE "TERAPIA")

## Beschreibung

Die vorliegende Erfindung betrifft ein Verfahren zur Herstellung von 3-Chlor-3'-nitro-4'-methoxybenzophenon mit einem sehr niedrigem Gehalt an Dinitroderivaten in der Rohware.

3-Chlor-3'-nitro-4'-methoxybenzophenon ist ein Zwischenprodukt, das zur Herstellung von pharmazeutischen Wirkstoffen verwendet werden kann (siehe z.B. EP-A-260744).

Es ist bekannt, 3-Chlor-3'-nitro-4'-methoxybenzophenon herzustellen, indem man 3-Chlor-4'-methoxybenzophenon in Methylenchlorid als Lösungsmittel mit einer Mischsäure nitriert oder die Nitrierung in Eisessig/Acetanhydrid als Lösungsmittel mit 65 gew.-%iger Salpetersäure bei 15°C durchführt (siehe Arzneimittel-Forschung 28/4, S. 586-594 (1978)).

Nachteilig bei diesen Verfahren ist, daß das rohe 3-Chlor-3'-nitro-4'-methoxybenzophenon mit einem Gehalt von 3 - 6 Gew.-% an Dinitroderivaten anfällt. Diese Dinitroderivate stören bei der weiteren Umsetzung zu pharmazeutischen Wirkstoffen und müssen daher entfernt werden. Mit üblichen Reinigungsmethoden, z.B. Umkristallisation aus einem Lösungsmittel, ist es allerdings nahezu unmöglich, diese Verunreinigungen zu entfernen, wenn sie zu über 4 Gew.-% in der Rohware enthalten sind. Auch geringere Gehalte an Dinitroderivaten in der Rohware erfordern Maßnahmen zur Reinigung, die mit hohen Substanzverlusten verbunden und deshalb unwirtschaftlich sind.

Im weiteren Verlauf der Synthese von pharmazeutischen Wirkstoffen können Dinitroderivate oder deren Folgeprodukte auch nicht auf befriedigende Weise abgetrennt werden.

Es stellt sich also die Aufgabe, ein Verfahren zur Herstellung von 3-Chlor-3'-nitro-4'-methoxybenzophenon zu finden, bei dem Dinitroderivate in deutlich kleinerer Menge als bisher gebildet werden.

Es wurde nun ein Verfahren zur Herstellung von 3-Chlor-3'-nitro-4'-methoxybenzophenon durch Nitrierung von 3-Chlor-4'-methoxybenzophenon gefunden, das dadurch gekennzeichnet ist, daß man die Nitrierung in 65 - 85 gew.-%iger Schwefelsäure als Reaktionsmedium mit Salpetersäure bei Temperaturen im Bereich von -20 bis +60°C durchführt.

Das Einhalten dieses Konzentrationsbereichs der Schwefelsäure ist wichtig, um eine gute Produktqualität zu erzielen. Die Gehalte an Dinitroderivaten bei so hergestellten Produkten liegen im allgemeinen bei weniger als 0,5 Gew.-%. Vorzugsweise setzt man in das erfindungsgemäße Verfahren 70 - 80 gew.-%ige Schwefelsäure ein.

Die Menge der Schwefelsäure, die der Nitrierung als Reaktionsmedium dient, kann in weiten Grenzen variiert werden. Sie wird vorzugsweise so bemessen, daß zu Beginn der Reaktion ein im wesentlichen homogenes Reaktionsgemisch entsteht und die Rührfähigkeit des nach Beendigung der Nitrierreaktion vorliegenden Reaktionsgemisches gewährleistet ist. Beispielsweise können 500 - 2 500 g Schwefelsäure pro 100 g eingesetztes 3-Chlor-4'-methoxybenzophenon verwendet werden. Vorzugsweise liegt diese Menge bei 1 000 - 1 800 g.

Die Konzentration der als Reaktand in das erfindungsgemäße Verfahren einzusetzenden Salpetersäure kann z.B. im Bereich 50 - 100 Gew.-% liegen. Bevorzugt ist 50 - 70 gew.-%ige Salpetersäure, insbesondere die als konzentrierte Salpetersäure handelsübliche ca. 63 gew.-%ige Ware. Die Salpetersäure kann als solche oder in beliebigen Mischungen mit Schwefelsäure eingesetzt werden. Falls man solche Salpetersäure/Schwefelsäure-Gemische einsetzt, können diese beispielsweise von 30 bis 60 Gew.-% Schwefelsäure (gerechnet als 100 gew.-%ige Säure, vorliegend als 96 bis 100 gew.-%ige Säure) und 40 bis 70 Gew.-% Salpetersäure der o.a. Konzentration enthalten.

Die Menge an Salpetersäure für das erfindungsgemäße Verfahren kann prinzipiell in weiten Bereichen variiert werden. Bevorzugt sind 80 - 150 Gew.-% der stöchiometrisch erforderlichen Menge. Eine geringere Menge an Salpetersäure führt zu unvollständigen Umsätzen und ist daher unwirtschaftlich, eine Erhöhung der Salpetersäuremenge über 150 Gew.-% der stöchiometrisch erforderlichen Menge hinaus fördert nicht die Selektivität der Reaktion und ist daher aus wirtschaftlichen Erwägungen wenig sinnvoll. Besonders bevorzugt wird bei dem erfindungsgemäßen Verfahren eine Menge Salpetersäure eingesetzt, die 90 - 120 Gew.-% der stöchiometrisch erforderlichen Menge entspricht.

Die Reaktionstemperatur beim erfindungsgemäßen Verfahren liegt bei -20 bis +60°C. Die Reaktionstemperatur ist nach unten durch das Nachlassen der Nitrierreaktion und der dann schlechten Löslichkeit des 3-Chlor-4'-methoxybenzophenons in der Schwefelsäure begrenzt. Temperaturen über 60°C hinaus führen häufig zu einem Nachlassen der Selektivität. Temperaturen im Bereich von 0 bis +40°C sind bevorzugt.

Das nach Beendigung der Nitrierreaktion und gegebenenfalls nach Ablauf einer Nachrührzeit vorliegende Reaktionsgemisch kann man z.B. Aufarbeiten, indem man den gebildeten Niederschlag abfiltriert, den Filterkuchen wäscht, z.B. mit Wasser und schließlich trocknet. Das so erhältliche Rohprodukt enthält weniger als 0,5 Gew.-% Dinitroderivate. Man kann das Rohprodukt gegebenenfalls weiter reinigen, z.B. durch Umlösung aus einem geeigneten Lösungsmittel. Das Rohprodukt wird im allgemeinen in Reinheiten von 93 % und höher erhalten. Das Reinprodukt kann in Ausbeuten von 85 % d.Th. und höher und mit Reinheiten von über 95 Gew.-% isoliert werden. Für die Umlösung geeignete Lösungsmittel sind z.B. Isopropanol, Diisopropylether und Toluol.

### Beispiele

Die nachfolgenden Beispiele sollen das erfindungsgemäße Verfahren erläutern, ohne es auf die dort beschriebenen Einzelheiten einzuschränken. Als Ausgangsprodukt wurde 3-Chlor-4'-methoxybenzophenon eingesetzt, das auf bekannte Weise durch Friedel-Crafts-Reaktion aus m-Chlorbenzoylchlorid und Anisol hergestellt worden war.

### Beispiel 1

In einer Rührapparatur wurden 175 ml 80 gew.-%ige Schwefelsäure vorgelegt und darin bei Raumtemperatur 22,2 g 3-Chlor-4'-methoxybenzophenon gelöst. Es resultierte eine klare gelbe Lösung. Dann wurde das Gemisch auf +10°C abgekühlt und innerhalb von 1 Stunde 10,5 g 65 gew.-%ige Salpetersäure zudosiert. Während der Zudosierung fiel die rohe Nitroverbindung aus der Reaktionslösung aus. Nach beendeter Dosierung wurde noch 1 Stunde bei +10°C nachgerührt und dann der gelbe Feststoff auf einer Glassinternutsche abfiltriert. Die Kristalle wurden mit Wasser nachgewaschen und getrocknet. Es resultierten 24,6 g Rohprodukt mit einer Reinheit von 96 Gew.-%. Das Rohprodukt enthielt 1,5 Gew.-% des Einsatzmaterials und einen Gehalt an Dinitroderivaten von unter 0,5 Gew.-%. Die Umlösung dieser Rohware aus 250 ml Isopropanol lieferte 23 g einer Ware mit einer Reinheit von über 99 Gew.-%. Das entspricht einer Ausbeute von 88 % der Theorie. Der Schmelzpunkt der gereinigten Ware betrug 113°C.

### Beispiel 2

Es wurde verfahren wie in Beispiel 1, jedoch wurde die Nitrierung in 175 ml 75 gew.-%iger Schwefelsäure bei +20°C durchgeführt und 11,5 g 65 gew.-%ige Salpetersäure eingesetzt. Nach der Isolierung durch Filtration, Waschen und Trocknen wurde ein Rohprodukt mit einer Reinheit von 94 Gew.-% erhalten, das 3,5 Gew.-% unumgesetztes Einsatzmaterial und weniger als 0,4 Gew.-% Dinitroderivate enthielt. Dieses Rohprodukt wurde auf die gleiche Weise wie in Beispiel 1 beschrieben, gereinigt und so eine über 99 Gew.-% reine Ware erhalten.

### Beispiel 3 (zum Vergleich)

In eine Mischung aus 43,3 g 3-Chlor-4'-methoxybenzophenon, 700 ml Dichlormethan und 44 ml konz. Schwefelsäure wurden bei +10 bis +15°C innerhalb von 15 Minuten 22 ml 65 gew.-%ige Salpetersäure zulaufen gelassen. Nach beendeter Dosierung wurde noch 1 Stunde bei 10°C nachgerührt. Nach der Zugabe von Wasser zu der rohen Nitrierlösung wurde die organische Phase abgetrennt, mit Natriumbicarbonat getrocknet und am Rotationsverdampfer eingedampft. Es verblieben 50 g eines Rohprodukts, das 93,9 Gew.-% 3-Chlor-3'-nitro-4'-methoxybenzophenon, 3,0 Gew.-% Dinitroderivate, 0,3 Gew.-% unumgesetztes Ausgangsprodukt und ergänzend zu 100 % unbekannte Anteile enthielt. Die Ausbeute an Rohprodukt betrug 92 % der Theorie.

## Patentansprüche

1. Verfahren zur Herstellung von 3-Chlor-3'-nitro-4'-methoxybenzophenon durch Nitrierung von 3-Chlor-4'-methoxybenzophenon, dadurch gekennzeichnet, daß man die Nitrierung in 65 bis 85 gew.-%iger Schwefelsäure als Reaktionsmedium mit Salpetersäure bei Temperaturen im Bereich von -20 bis +60°C durchführt.

2. Verfahren nach Anspruch 1, dadurch gekennzeichnet, daß man 70 bis 80 gew.-%ige Schwefelsäure einsetzt.

3. Verfahren nach Ansprüchen 1 und 2, dadurch gekennzeichnet, daß man, bezogen auf 100 g 3-Chlor-4'-methoxybenzophenon, 500 bis 2 500 g Schwefelsäure einsetzt.

4. Verfahren nach Ansprüchen 1 bis 3, dadurch gekennzeichnet, daß Salpetersäure einer Konzentration im Bereich 50 bis 100 Gew.-% in einer Menge eingesetzt wird, die 80 bis 150 Gew.-% der stöchiometrisch erforderlichen Menge entspricht.

5. Verfahren nach Ansprüchen 1 bis 4, dadurch gekennzeichnet, daß man die Nitrierung bei 0 bis +40°C durchführt.

6. Verfahren nach Ansprüchen 1 bis 5, dadurch gekennzeichnet, daß man das nach Beendigung der Nitrierreaktion und gegebenenfalls nach Ablauf einer Nachrührzeit vorliegende Reaktionsgemisch aufarbeitet, indem man den gebildeten Niederschlag abfiltriert, den Filterkuchen wäscht und trocknet.

7. Verfahren nach Anspruch 6, dadurch gekennzeichnet, daß man das nach Anspruch 6 erhaltene Rohprodukt durch Umlösung aus einem geeigneten Lösungsmittel weiter reinigt.

## Claims

1. Process for the preparation of 3-chloro-3'-nitro-4'-methoxbenzophenone by nitrating 3-chloro-4'-methoxybenzophenone, characterized in that the nitration is carried out in from 65 to 85 % strength by weight sulphuric acid as reaction medium with nitric acid at temperatures in the range from -20 to +60EC.

2. Process according to Claim 1, characterized in that from 70 to 80 % strength by weight sulphuric acid is employed.

3. Process according to Claims 1 and 2, characterized in that from 500 to 2500 g of sulphuric acid are employed, based on 100 g of 3-chloro-4'-methoxybenzophenone.

4. Process according to Claims 1 to 3, characterized in that nitric acid with a concentration in the range from 50 to 100 % by weight is employed in an amount which corresponds to from 80 to 150 % by weight of the stoichiometrically required amount.

5. Process according to Claims 1 to 4, characterized in that the nitration is carried out at from 0 to +40°C.

6. Process according to Claims 1 to 5, characterized in that the reaction mixture present at the end of the nitration reaction and, if desired, after the conclusion of an after-stirring period is worked up by filtering off the precipitated formed, washing the filter cake and drying it.

7. Process according to Claim 6, characterized in that the crude product obtained in accordance with Claim 6 is purified further by reprecipitation from an appropriate solvent.

## Revendications

1. Procédé pour la préparation de la 3-chloro-3'-nitro-4'-méthoxybenzophénone par nitration de la 3-chloro-4'-méthoxybenzophénone, caractérisé en ce qu'on effectue la nitration dans de l'acide sulfurique à 65 à 85% en poids à titre de milieu de réaction avec de l'acide nitrique à des températures dans le domaine de -20 à +60°C.

2. Procédé selon la revendication 1, caractérisé en ce qu'on met en oeuvre l'acide sulfurique à 70 à 80% en poids.

3. Procédé selon les revendications 1 et 2, caractérisé en ce qu'on met en oeuvre, rapportés à 100 g de la 3-chloro-4'-méthoxybenzophénone, de 500 à 2500 g d'acide sulfurique.

4. Procédé selon les revendications 1 à 3, caractérisé en ce qu'on met en oeuvre de l'acide nitrique de concentration comprise dans le domaine de 50 à 100% en poids, en une quantité qui constitue de 80 à 150% en poids de la quantité requise du point de vue stoechiométrique.

5. Procédé selon les revendications 1 à 4, caractérisé en ce qu'on effectue la nitration à une température de 0 à +40°C.

6. Procédé selon les revendications 1 à 5, caractérisé en ce qu'on traite le mélange réactionnel que l'on obtient au terme de la réaction de nitration et le cas échéant après achèvement d'un temps d'agitation ultérieur par le fait que l'on sépare par filtration le précipité obtenu, on lave et on sèche le gâteau de filtre.

7. Procédé selon la revendication 6, caractérisé en ce qu'on soumet le produit brut obtenu conformément à la revendication 6 à une purification ultérieure par recristallisation dans un solvant approprié.
